(19) Europäisches Patentamt
European Patent Office
Office européen des brevets

(11) Veröffentlichungsnummer: **0 204 269**
**B1**

# (12) EUROPÄISCHE PATENTSCHRIFT

(45) Veröffentlichungstag der Patentschrift: **02.05.90**

(51) Int. Cl.⁴: **C07D 213/66**, C07D 491/048, A61K 31/44

(21) Anmeldenummer: **86107333.6**

(22) Anmeldetag: **30.05.86**

## (54) Pyridin-Derivate, ihre Herstellung und Verwendung.

(30) Priorität: **01.06.85 DE 3519693**

(43) Veröffentlichungstag der Anmeldung:
**10.12.86 Patentblatt 86/50**

(45) Bekanntmachung des Hinweises auf die Patenterteilung:
**02.05.90 Patentblatt 90/18**

(84) Benannte Vertragsstaaten:
**AT BE CH DE FR GB IT LI NL SE**

(56) Entgegenhaltungen:
**EP-A- 0 040 591**
**DE-A- 2 711 655**
**DE-A- 3 204 596**

(73) Patentinhaber: **BASF Aktiengesellschaft, Carl-Bosch-Strasse 38, D-6700 Ludwigshafen(DE)**

(72) Erfinder: **Hege, Hans-Guenther, Dinckelackerring 29, D-6730 Neustadt 14(DE)**
Erfinder: **Eisen, Gerhard, Schillerstrasse 5, D-6707 Schifferstadt(DE)**
Erfinder: **Koenig, Horst, Prof. Dr., Pariser Strasse 4, D-6700 Ludwigshafen(DE)**
Erfinder: **von Philipsborn, Gerda, Dr., Naechstenbacher Weg 35, D-6940 Weinheim(DE)**

EP 0 204 269 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Pyridin-Derivate, Verfahren zu deren Herstellung sowie deren Verwendung bei der Bekämpfung von Krankheiten.

In der DE-OS 2 711 655 sind bereits Pyridinyl-aminoalkylether beschrieben, die antiarrhythmische und/oder lokalanästhetische Wirkungen besitzen. Die bekannteste dieser Verbindungen ist das Barucainid (Beispiel 34 der genannten DE-OS).

Es wurden nun Verbindungen mit einer überlegenen Wirkung gefunden.

Gegenstand der Erfindung sind Pyridin-Derivate der Formel I

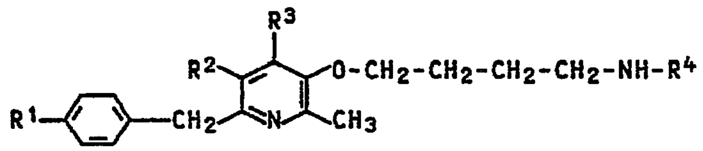

I,

worin

$R^1$ ein Wasserstoffatom oder eine Hydroxygruppe,

$R^2$ und $R^3$ zusammen die Gruppe $-CH_2-O-CH_2-$, worin ein Wasserstoffatom durch eine Hydroxy- oder Methoxygruppe ersetzt ist, und

$R^4$ eine n-Propyl-, Isopropyl- oder n-Butylgruppe darstellen, sowie deren Salze mit physiologisch verträglichen Säuren.

Bevorzugt sind die Verbindungen der Formel I, in der $R^1$ die angegebene Bedeutung hat, sowie $R^2$ und $R^3$ zusammen die Gruppen $-CHOH-O-CH_2$ oder $-CH_2-O-CHOH-$ und $R^4$ eine Isopropylgruppe darstellen.

Als physiologisch verträgliche Säuren eignen sich insbesondere zur Salzbildung:
Halogenwasserstoffsäuren, Schwefelsäure, Phosphorsäure, Salpetersäure, Fumarsäure, aliphatische, alicyclische, aromatische oder heterocyclische Carbon- oder Sulfonsäuren, wie Ameisensäure, Essigsäure, Propionsäure, Bernsteinsäure, Glykolsäure, Milchsäure, Äpfelsäure, Weinsäure, Zitronensäure, Ascorbinsäure, Maleinsäure, Brenztraubensäure, Benzoesäure, Anthranilsäure, p-Hydroxybenzoesäure oder Salicylsäure, Methansulfonsäure, Ethansulfonsäure, Hydroxyethansulfonsäure, Ethylensulfonsäure, Halogenbenzolsulfonsäure, Toluolsulfonsäure, Cyclohexylaminosulfonsäure oder Sulfanilsäure. Weitere Säuren können aus Fortschritte der Arzneimittelforschung, Band 10, Seite 224 bis 225, Birkhäuser-Verlag, Basel und Stuttgart, 1966, entnommen werden.

Die Verbindungen der Formel I lassen sich herstellen, indem man

a) ein Pyridinol der Formel II

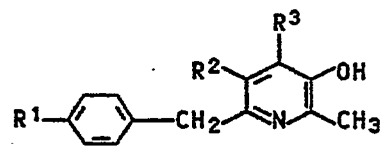

II,

worin $R^1$, $R^2$ und $R^3$ die angegebene Bedeutung besitzen, mit einer Verbindung der Formel III

$X-CH_2-CH_2-CH_2-CH_2-NHR^4$ III,

worin $R^4$ die angegebene Bedeutung hat und X eine reaktionsfähige Gruppe bedeutet, oder

b) ein Pyridinol der Formel II mit einer Verbindung der Formel IV

$Y-CH_2-CH_2-CH_2-CH_2-Z$ IV,

worin Y und Z ein Halogenatom bedeuten, und anschließend mit einem Anion der Formel V

$NH_2-R^4$ V,

worin $R^4$ die angegebene Bedeutung besitzt, oder

c) ein Amin der Formel VI

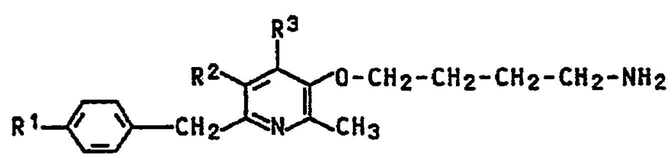

VI,

worin $R^1$, $R^2$ und $R^3$ die angegebene Bedeutung haben, mit einer Verbindung der Formel VII

$X-R^4$ VII,

worin X und $R^4$ die angegebenen Bedeutungen besitzen, umsetzt.

Die Verbindungen der Formel I, in denen $R^2$ und $R^3$ zusammen die Gruppe $-CH_2-O-CH_2$ bedeuten,

worin ein Wasserstoffatom durch eine Hydroxygruppe ersetzt ist, werden erhalten, indem man eine Verbindung der Formel VIIIa oder VIIIb

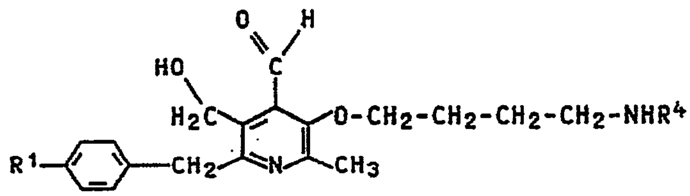

VIIIa,

oder

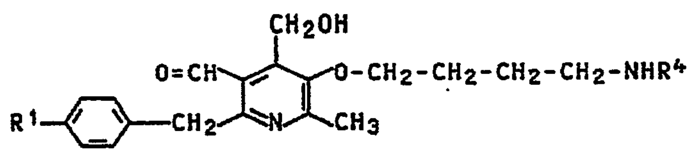

VIIIb,

worin $R^1$ und $R^4$ die angegebene Bedeutungen besitzen, in Gegenwart einer Säure umsetzt. Die so erhaltenen Verbindungen werden gegebenenfalls in ihre Salze mit physiologisch verträglichen Säuren überführt.

Die Umsetzung a) wird zweckmäßigerweise in einem Lösungsmittel durchgeführt bei Temperaturen von 0 bis 150°C, vorzugsweise 20 bis 100°C. Zweckmäßige Lösungsmittel sind niedere Alkohole mit 1 bis 4 C-Atomen, insbesondere Methanol oder Ethanol, niedere aliphatische Ketone, insbesondere Aceton, Benzol oder Alkyl- oder Halogenbenzole, wie Chlorbenzol oder Toluol, aliphatische oder cyclische Ether, wie Diethylether, Tetrahydrofuran oder Dioxan, Dimethylformamid oder Dimethylsulfoxid. Wenn ein Ether als Lösungsmittel verwendet wird, kann diesem zweckmäßig als zusätzliches Lösungsmittel Hexamethylphosphorsäuretriamid zugesetzt werden.

In einer vorteilhaften Variante, insbesondere dann, wenn keine leicht verseifbaren funktionellen Gruppen im Pyridinol vorhanden sind, werden zweiphasige Lösungsmittelgemische, insbesondere Mischungen von Wasser mit einem chlorierten Kohlenwasserstoff, wie Dichlormethan, oder einem Benzolkohlenwasserstoff, wie Benzol oder Toluol, verwendet und dabei die an sich bekannte Methodik der Phasentransfer-Katalyse, wie beispielsweise von M. Makosza in Pure and Applied Chemistry, 1975, Nr. 43, Seite 439 beschrieben, angewandt. Die bevorzugten Basen sind hierbei Basengemische aus einem Alkalihydroxid, insbesondere Natriumhydroxid, und einer quartären Ammoniumbase oder einer Phosphoniumbase, die in katalytischer Menge in Form ihres Salzes eingesetzt wird, beispielsweise Triethylbenzylammoniumchlorid, Tetrabutylammonium-hydrogensulfat, Tributyl-hexadecylphosphoniumbromid.

Als reaktionsfähige Gruppe für X ist insbesondere eine durch eine starke anorganische oder organische Säure, vor allem eine Halogenwasserstoffsäure, wie Chlorwasserstoffsäure, Bromwasserstoffsäure oder Iodwasserstoffsäure, Schwefelsäure oder eine starke organische Sulfonsäure, wie beispielsweise Benzolsulfonsäure, Methansulfonsäure oder 4-Toluolsulfonsäure, veresterte Hydroxygruppe zu nennen: Dabei ist für X Chlor, Brom oder Jod bevorzugt.

Die Umsetzung wird zweckmäßig in Gegenwart einer äquivalenten oder überschüssigen Menge Base als säurebindendes Mittel, wie einem Alkalihydroxid, -carbonat oder -alkoholat, wobei insbesondere die entsprechenden Natrium- oder Kaliumverbindungen verwendet werden, durchgeführt.

Für die Umsetzung kann man auch die Verbindung II in Form ihres Alkalisalzes, insbesondere des Natrium- oder Kaliumsalzes verwenden. Zur Salzbildung werden die oben genannten Alkaliverbindungen verwendet oder auch, insbesondere wenn ein aprotisches Lösungsmittel verwendet wird, Natrium -oder Kaliumamid oder -hydrid.

Die Verfahrensbedingungen für die Umsetzung von II mit IV (Verfahren b)) entsprechen im Hinblick auf die verwendeten Lösungsmittel, Basen als säurebindende Mittel und Temperaturen den im Verfahren a) genannten. Um die Bildung von Nebenprodukten, insbesondere Veretherungen aus 2 Mol II möglichst niedrig zu halten, wird vorteilhafterweise die Verbindung IV in wenigstens doppelt molarem Überschuß verwendet oder man verwendet eine Verbindung, in der X und Y verschieden sind, so daß deren unterschiedliche Reaktivität ausgenutzt werden kann, wie es beispielsweise bei Brom gegenüber Chlor der Fall ist.

Das so entstehende Zwischenprodukt der Formel IX

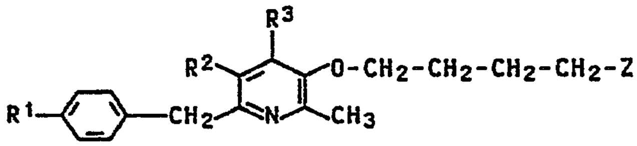

IX,

kann isoliert und anschließend für sich mit einem Amin $NH_2R^4$ umgesetzt werden oder auch direkt in dem erhaltenen Reaktionsgemisch aus dem ersten Verfahrensschritt mit dem Amin umgesetzt werden.

Diese Umsetzung wird ebenso, wie oben angegeben, zweckmäßig in einem Lösungsmittel und in Gegenwart einer Base durchgeführt. Dabei kann als Base auch ein Überschuß des Amins $NH_2–R^4$ verwendet werden, das auch gleichzeitig als Lösungsmittel dienen kann. Die Umsetzung erfolgt bei erhöhten Temperaturen, im allgemeinen bei Temperaturen von 60 bis 20°C unter Normaldruck oder gegebenenfalls in einem geschlossenen Gefäß unter erhöhtem Druck, besonders wenn ein leichtflüchtiges Amin verwendet wird.

Die Aminalkylierung gemäß Verfahren c) wird zweckmäßigerweise in einem niederen Alkohol, bevorzugt Methanol oder Ethanol, und in Gegenwart einer Base als säurebindendes Mittel, bevorzugt Natriumcarbonat, bei Raumtemperaturen oder erhöhten Temperaturen bis zu Rückflußtemperaturen durchgeführt.

Die Herstellung einer Verbindung der Formel I, in der $R^4$ eine Isopropylgruppe bedeutet, kann auch durch reduktive Aminalkylierung erfolgen. Diese Umsetzung wird vorzugsweise als katalytische Hydrierung in Gegenwart von Platin durchgeführt.

Die für die Herstellungsverfahren benötigten Ausgangsstoffe sind nach bekannten Verfahren erhältlich (vgl. beispielsweise DE-OS 2 711 655).

Die Verbindungen der Formel VI können durch Erhitzen der Verbindungen IX, gelöst in einem niederen Alkohol, wie Ethanol, mit überschüssigem Ammoniak in einem geschlossenen System hergestellt werden. Sie können auch durch Erhitzen von Verbindungen der Formel IX, gelöst in einem polaren Lösungsmittel, insbesondere Dimethylformamid, mit Phthalimidkalium unter Bildung der entsprechenden Phthalimidderivate und deren Umsetzung mit Hydroxylamin, vorzugsweise in einem niederen Alkohol, wie Methanol, und in Gegenwart eines Alkoholats, wie Natriummethanolat, hergestellt werden.

Je nach den Verfahrensbedingungen und Ausgangsverbindungen erhält man die erfindungsgemäßen Verbindungen in freier Form oder in der ebenfalls in der Erfindung inbegriffenen Form ihrer Säureadditionssalze. Die Säureadditionssalze können als basische, neutrale oder gemischte Salze, gegebenenfalls in Form von Hydraten vorliegen. Die beim Herstellungsverfahren erhaltenen Säureadditionssalze können in an sich bekannter Weise in die freie Base übergeführt werden, mit basischen Mitteln, wie Alkali- oder Ionenaustauschern. Andererseits können die erhaltenen freien Basen ohne weiteres mit organischen oder anorganischen Säuren in die Salze übergeführt werden.

Gegebenenfalls können die Säureadditonssalze, wie die Pikrate oder Perchlorate, auch zur Reinigung der erhaltenen Verbindungen dienen, indem man die freien Basen in diese Salze überführt, diese abtrennt und aus den Salzen wiederum die Basen freisetzt.

Die neuen Verbindungen können zum Teil je nach der Wahl der Ausgangsverbindungen und Arbeitsweisen als optische Antipoden oder Racemate anfallen. Erhaltene Racemate lassen sich nach bekannten Methoden in die optischen Antipoden zerlegen, beispielsweise durch Umsetzen mit einer, mit der racemischen Verbindung Salze bildenden optisch aktiven Säure und Trennung in die Diastereomeren, oder mit Hilfe von Mikroorganismen oder durch Umkristallisation aus einem optisch aktiven Lösungsmittel. Besonders gebräuchliche optisch aktive Säuren sind z.B. die D- und L-Formen von Weinsäure, Äpfelsäure, Mandelsäure oder Camphersulfonsäure.

Die erfindungsgemäßen Verbindungen sind stark antiarrhythmisch wirksam und eignen sich zur Therapie von Herzrhythmusstörungen. Die pharmakologischen Untersuchungen zur Bestimmung der antiarrhythmischen Wirkung wurden im Vergleich zu dem Antiarrhythmikum Barucainid durchgeführt.

Als Versuchstiere dienten männliche Sprague-Dawley-Ratten im Gewicht von 180 bis 300 g. Die Narkose erfolgte mit 100 mg/kg Thiobutabarbital i.p. Zur Erzeugung von Arrhythmien wurde Akonitin mit einer Dosierungsgeschwindigkeit von 5 $\mu g/kg \cdot min^{-1}$ infundiert. Die Prüfsubstanzen wurden intravenös unmittelbar vor Beginn der Akonitin-Infusion appliziert. Meßgröße ist die Menge Akonitin, die bei den Tieren im EKG zum Auftreten der ersten Arrhythmien (Verlust von P, ventrikuläre Extrasystolen und Tachykardien) führt. Bei unbehandelten Tieren betrug die arrhythmogene Akonitin-Dosis 16,5±0,34 µg/kg (n=120). Aus der linearen Beziehung zwischen log Dosis (mg/kg) der Prüfsubstanzen und der relativen Zunahme der arrhythmogenen Akonitin-Dosis (Δ %) wird die Dosis bestimmt, die 50% Zunahme bewirkt.

Als weiteres Kriterium für die antiarrhythmische Wirkstärke wird die maximal erreichte Wirkung (Δ %) gewertet.

Außerdem wird die Dosis der Prüfsubstanz ermittelt, die im EKG toxische Veränderungen hervorruft (ST-Senkung; P-Verlust; Extrasystolen). Als Maß für die therapeutische Breite der neuen Verbindungen wird der Quotient aus der EKG-toxischen Dosis und der antiarrhythmischen ED 50 gewertet.

Die Tabelle zeigt die antiarrhythmische Wirkung (ED 50), die maximal erreichte Wirkung (Δ %) und die therapeutische Breite der neuen Verbindungen.

Beim Vergleich der ED 50-Werte haben die Substanzen der Beispiele 1, 2 und 3 eine 2,8- bis 10,3fach stärkere Wirkung als Barucainid. Weiterhin übertreffen die Substanzen der Beispiele 2, 3 und 4 Barucainid hinsichtlich der maximal erreichten Wirkung. Die therapeutische Breite ist bei allen Substanzen deutlich größer als bei Barucainid.

Tabelle 1

Antiarrhythmische Wirkung und therapeutische Breite bei der Ratte

| Beispiel Nr. | Antiarrhythmische Wirkung[1] ED 50 (mg/kg) | Maximaler Effekt (Δ %) | EKG-toxische[2] Dosis (mg/kg) | Therapeutische Breite:[3] |
|---|---|---|---|---|
| Barucainid | 1,95 | 105 | 10,0 | 5,13 |
| 1 | 0,468 | 239 | >4,64 | >9,9 |
| 2 | 0,695 | 276 | 10 | 14,4 |

[1] Antiarrhythmische Wirkung auf die Akonitinarrhythmie (Zunahme der Dauer der Akonitininfusion bis zum Auftreten von Arrhythmien)

[2] Toxische Wirkung auf das EKG (Auftreten von Extrasystolen)

[3] Therapeutische Breite (toxische Dosis: ED 50)

Die therapeutischen Mittel bzw. Zubereitungen werden mit den üblichen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder Depotformen.

Selbstverständlich kommen auch parenterale Zubereitungsformen, wie Injektionslösungen oder Zusätze zu Infusionslösungen, in Betracht. Weiterhin seien als Zubereitungen beispielsweise auch Suppositorien genannt.

Die entsprechenden Tabletten können beispielsweise durch Mischen des Wirkstoffs mit bekannten Hilfsstoffen, beispielsweise inerte Verdünnungsmittel, wie Dextrose, Zucker, Sorbit, Mannit, Polyvinylpyrrolidon, Calciumcarbonat, Calciumphosphat oder Milchzucker, Sprengmitteln, wie Mais, Stärke, Alginsäure oder Polyvinylpyrrolidon, Bindemitteln, wie Stärke oder Gelatine, Gleitmitteln, wie Magnesiumstearat oder Talkum, und/oder Mitteln zur Erzielung des Depoteffektes, wie Carboxypolymethylen, Carboxymethylcellulose, Celluloseacetatphthalat oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit den erfindungsgemäßen Wirkstoffen können zusätzlich geschmackverbessernde Mittel, wie Saccharin, Cyclamat oder Zucker sowie beispielsweise Aromastoffe, wie Vanillin oder Orangenextrakt, enthalten. Sie können außerdem Suspendierhilfsstoffe, wie Natriumcarboxymethylcellulose, oder Konservierungsstoffe, wie Parahydroxybenzoate, enthalten. Wirkstoffe enthaltende Kapseln können beispielsweise hergestellt werden, indem man den Wirkstoff mit einem inerten Träger, wie Milchzucker oder Sorbit, mischt und in Gelatinekapseln einkapselt.

Geeignete Suppositorien lassen sich beispielsweise durch Vermischen mit dafür vorgesehenem Trägermaterial, wie Neutralfetten oder Polyethylenglykolen bzw. dessen Derivaten, herstellen.

Die Einzeldosis einer erfindungsgemäßen Substanz am Menschen liegt bei 5 bis 100 mg, vorzugsweise bei 10 bis 80 mg.

Die folgenden Beispiele erläutern die Erfindung, ohne sie jedoch einzuschränken.

Beispiel 1

1-Hydroxy-4-benzyl-6-methyl-7-(4-isopropylaminobutoxy)-1,3-dihydrofuro-[3,4-C]-pyridin-dihydrochlorid

A. Herstellung des Ausgangsmaterials

30,0 g (0,115 Mol) 6-Benzylpyridoxin wurden in 1,5 l Wasser und 3,5 ml 4N Schwefelsäure gelöst. Man leitete Stickstoff durch die Lösung und trug 170 g (1,95 Mol) Mangandioxid portionsweise im Verlauf von 30 min ein. Man rührte 3,5 h bei Raumtemperatur weiter, filtrierte das anorganische Material ab und wäscht den Rückstand mit Wasser. Nach Einengen des Filtrats am Rotationsverdampfer nahm man in 500 ml Methanol auf und saugte erneut ab. Das gelbe Filtrat wurde vollständig eingeengt und in 2 l Methanol gelöst. Nach 2 Tagen bei Raumtemperatur engte man ein und erhielt 21 g Rückstand, der mit Natriumhydrogencarbonat auf pH 8,5 gestellt wurde. Der Festkörper wurde abgesaugt und aus Methanol umkristallisiert. Man erhielt 14,0 g 1-Methoxy-4-benzyl-6-methyl-7-hydroxy-1,3-dihydrofuran-[3,4-C]-pyridin als gelbe Nadeln, Fp. 177 bis 178°C (Zersetzung).

Das so erhaltene Produkt wurde in die entsprechende 4-Brombutoxy-Verbindung übergeführt.

Dazu löste man 10 g in 500 ml Aceton, versetzte mit 43 g 1,4-Dibrombutan und 110 g $K_2CO_3$. Man erwärmte 5 h zum Rückfluß, saugte ab, wusch den Rückstand mit Aceton nach und engte unter Vakuum, zuletzt bei 60°C ein. Man erhielt 16 g bräunliches Öl.

Durch Lösen in 50 ml Isopropylamin bei 25°C erhielt man nach 48 h 1-Methoxy-4-benzyl-6-methyl-7-(4-isopropylaminobutoxy)-1,3-dihydrofuro-[3,4-C]-pyridin in Form eines braunen Harzes nach Abdestillieren des Amin-Überschusses.

B. Herstellung des Endprodukts

Ohne weitere Reinigung wurde das gemäß A erhaltene Produkt in 100 ml HCl 16 h bei Raumtemperatur gehalten, nach Zusatz von 2 g Aktivkohle 1 h gerührt und filtriert. Man wusch das Papierfilter mit Wasser nach und engte unter Vakuum bei 50°C auf 50 ml ein. Man sättigte mit Ethylenglykol-dimethylether und filtrierte nach 20 h Stehen bei 20°C. Durch Waschen mit $H_2O$-Ethylenglykol-dimethylether 1:5 und zuletzt mit dem reinen Lösungsmittel erhielt man 9 g kristallines 1-Hydroxy-4-benzyl-6-methyl-7-(4-isopropylaminobutoxy)-1,3-dihydrofuro-[3,4-C]-pyridin-dihydrochlorid, Fp. 194 bis 197°C.

Beispiel 2

3-Hydroxy-4-benzyl-6-methyl-7-(4-isopropylaminobutoxy)-1,3-dihydrofuro-[3,4-C]-pyridin

A. Herstellung des Ausgangsmaterials

a) 6-Benzylpyridoxin wurden unter Kühlung im Eisbad und gutem Rühren mit Schwefelsäure und Aceton in Analogie zu H. Cohen, E.G. Hughes, J. Chem. Soc. 4384 (1952) in das Acetonid umgewandelt. Dabei entstand ein Isomeren-Gemisch, das überwiegend das gewünschte $\alpha^4,\alpha^5$-6-Ring-Acetal zur phenolischen Hydroxylgruppe enthielt. Man neutralisierte den Ansatz unter gutem Kühlen und guter Rührung durch Eingießen in überschüssige wäßrige Natronlauge, die mit Essigsäureethylester überschichtet war. Extraktion der wäßrigen Phase mit mehreren Portionen Essigester, Waschen mit 2N NaOH und $H_2O$, Trocknen über $K_2CO_3$, Filtrieren und Einengen bei 40°C am Rotationsverdampfer lieferten ein hellbraunes Öl. Aus Petrolether kristallisierte das reine gewünschte Acetonid, Fp. 120 bis 121°C (farblose Nadeln).

b) 13 g dieses Produktes wurden in 1500 ml Aceton gelöst und auf 5°C gekühlt. Man versetzte mit 200 g Mangandioxid und erhielt nach 20 h in 95% Ausbeute $\alpha^4$,3-O-Isopropyliden-6-benzyl-pyridoxal aus der Acetonphase durch Filtrieren und Einengen des Filtrats.

o) Das so erhaltene Produkt wurde ohne weitere Reinigung in 60 ml 2N Salzsäure gelöst. Es entstand sehr rasch ein beiges Kristallisat. Durch Rühren bei 50°C brachte man den Feststoff in Lösung, nach 3 h war das Acetonid völlig verseift. Man engte am Rotationsverdampfer bei 50°C ein, nahm in 1500 ml Methanol auf, erhitzte 17 h zum Rückfluß und engte erneut ein. Man erhielt 3-Methoxy-4-benzyl-6-methyl-7-hydroxy-1,3-dihydrofuro-[3,4-C]-pyridin.

d) Das so erhaltene Produkt wurde in 1000 ml Aceton gelöst, mit 100 g Kaliumcarbonat und 90 g 1,4-Dibrombutan 15 h unter Rückfluß gerührt. Man saugte ab und wusch den Rückstand gut mit Aceton. Nach Einengen und Abdestillieren des überschüssigen 1,4-Dibrombutans im Hochvakuum bei 50°C erhielt man 17 g hellbraunes, zähflüssiges Öl.

e) Das gemäß d) erhaltene Produkt wurde ohne weitere Reinigung in 100 ml Isopropylamin gelöst und 6 h bei Raumtemperatur gehalten. Man engte erneut ein, löste in Methanol und entfernte das Lösungsmittel und restliches Isopropylamin im Vakuum. Der Eindampfrückstand von 29 g braunem Öl wurde in 10 ml 5N NaOH und 50 ml Methyl-tert.-butylether verteilt. Man wusch die Alkaliphase mit Methyl-tert.-butylether nach und trocknete die organische Phase über Kaliumcarbonat. Nach Einengen erhielt man aus der organischen Lösung 17 g hellbraunes Harz.

B. Herstellung des Endprodukts

Das gemäß A.e) erhaltene Produkt wurde 17 h bei Zimmertemperatur in 100 ml 2N HCl und 200 ml $H_2O$ gerührt. Man engte bei 50°C am Rotationsverdampfer ein, gab zum Rückstand Ethylenglykoldimethylether und ließ 2 Tage bei 3°C stehen. Das Kristallisat wurde abgesaugt, mit Wasser-Glykolether gewaschen und getrocknet. Man erhielt 7 g 3-Hydroxy-4-benzyl-6-methyl-7-(4-isopropylaminobutoxy)-1,3-dihydrofuro-[3,4-C]-pyridin-dihydrochlorid, Fp. 188°C.

Analog lassen sich herstellen:

1-Hydroxy-4-benzyl-6-methyl-7-(4-n-propylaminobutoxy)-1,3-dihydrofuro-[3,4-C]-pyridindihydrochlorid,
1-Hydroxy-4-benzyl-6-methyl-7-(4-n-butylaminobutoxy)-1,3-dihydrofuro-[3,4-C]-pyridindihydrochlorid,
1-Hydroxy-4-benzyl-6-methyl-7-(4-isobutylaminobutoxy)-1,3-dihydrofuro-[3,4-C]-pyridindihydrochlorid,
1-Methoxy-4-benzyl-6-methyl-7-(4-n-propylaminobutoxy)-1,3-dihydrofuro-[3,4-C]-pyridindihydrochlorid,
1-Methoxy-4-benzyl-6-methyl-7-(4-n-butylaminobutoxy)-1,3-dihydrofuro-[3,4-C]-pyridindihydrochlorid,
1-Methoxy-4-benzyl-6-methyl-7-(4-isobutylaminobutoxy)-1,3-dihydrofuro-[3,4-C]-pyridindihydrochlorid,
1-Methoxy-4-benzyl-6-methyl-7-(4-isopropylaminobutoxy)-1,3-dihydrofuro-[3,4-C]-pyridindihydrochlorid,
3-Methoxy-4-benzyl-6-methyl-7-(4-isopropylaminobutoxy)-1,3-dihydrofuro-[3,4-C]-pyridin,
3-Methoxy-4-benzyl-6-methyl-7-(4-n-propylaminobutoxy)-1,3-dihydrofuro-[3,4-C]-pyridin,
3-Methoxy-4-benzyl-6-methyl-7-(4-n-butylaminobutoxy)-1,3-dihydrofuro-[3,4-C]-pyridin,
3-Methoxy-4-benzyl-6-methyl-7-(4-isobutylaminobutoxy)-1,3-dihydrofuro-[3,4-C]-pyridin,
3-Hydroxy-4-benzyl-6-methyl-7-(4-isopropylaminobutoxy)-1,3-dihydrofuro-[3,4-C]-pyridin,
3-Hydroxy-4-benzyl-6-methyl-7-(4-n-propylaminobutoxy)-1,3-dihydrofuro-[3,4-C]-pyridin,
3-Hydroxy-4-benzyl-6-methyl-7-(4-n-butylaminobutoxy)-1,3-dihydrofuro-[3,4-C]-pyridin,
3-Hydroxy-4-benzyl-6-methyl-7-(4-isobutylaminobutoxy)-1,3-dihydrofuro-[3,4-C]-pyridin.

**Patentansprüche für die Vertragsstaaten: BE, CH, DE, FR, GB, IT, LI, NL, SE**

1. Pyridin-Derivate der Formel I

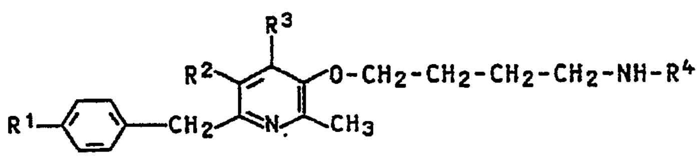

I,

worin
$R^1$ ein Wasserstoffatom oder eine Hydroxygruppe,
$R^2$ und $R^3$ zusammen die Gruppe $-CH_2-O-CH_2-$, worin ein Wasserstoffatom durch eine Hydroxy- oder Methoxygruppe ersetzt ist, und
$R^4$ eine n-Propyl-, Isopropyl- oder n-Butylgruppe
darstellen, sowie deren Salze mit physiologisch verträglichen Säuren.

2. 1-Hydroxy-4-benzyl-6-methyl-7-(4-isopropylaminobutoxy)-1,3-dihydrofuro-[3,4-C]-pyridin-dihydrochlorid.

3. 3-Hydroxy-4-benzyl-6-methyl-7-(4-isopropylaminobutoxy)-1,3-dihydrofuro-[3,4-C]-pyridin.

4. Verbindungen der Formel I gemäß Anspruch 1 zur Verwendung bei der Bekämpfung von Krankheiten.

5. Verwendung der Verbindungen der Formel I zur Herstellung von Arzneimitteln mit antiarrhythmischer Wirkung.

**Patentansprüche für den Vertragsstaat: AT**

1. Verfahren zur Herstellung der Pyridin-Derivate der Formel I

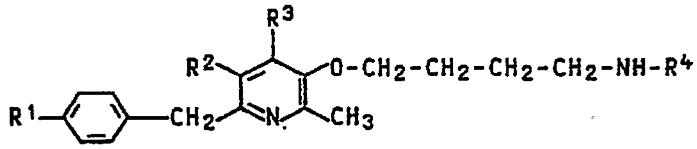

I,

worin
$R^1$ ein Wasserstoffatom oder eine Hydroxygruppe,
$R^2$ und $R^3$ zusammen die Gruppe $-CH_2-O-CH_2-$, worin ein Wasserstoffatom durch eine Hydroxy- oder Methoxygruppe ersetzt ist, und
$R^4$ eine n-Propyl-, Isopropyl- oder n-Butylgruppe
darstellen, sowie deren Salze mit physiologisch verträglichen Säuren, dadurch gekennzeichnet, daß man
a) ein Pyridinol der Formel II

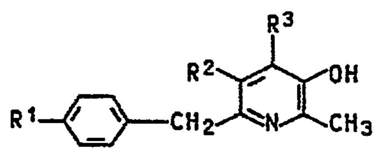

II,

worin $R^1$, $R^2$ und $R^3$ die angegebene Bedeutung besitzen, mit einer Verbindung der Formel III
$X-CH_2-CH_2-CH_2-CH_2-NHR^4$ III,
worin $R^4$ die angegebene Bedeutung hat und X eine reaktionsfähige Gruppe bedeutet, oder
b) ein Pyridinol der Formel II mit einer Verbindung der Formel IV
$Y-CH_2-CH_2-CH_2-CH_2-Z$ IV,
worin Y und Z ein Halogenatom bedeuten, und anschließend mit einem Amin der Formel V
$NH_2-R^4$ V,
worin $R^4$ die angegebene Bedeutung besitzt, oder
c) ein Amin der Formel VI

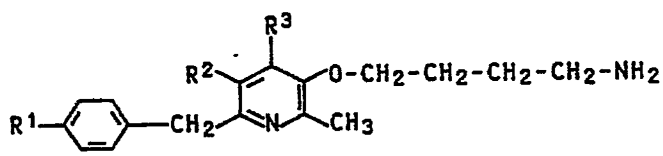

VI,

worin $R^1$, $R^2$ und $R^3$ die angegebene Bedeutung haben, mit einer Verbindung der Formel VII
$X-R^4$ VII,
worin X und $R^4$ die angegebene Bedeutungen besitzen, umsetzt und die so erhaltenen Verbindungen gegebenenfalls in ihre Salze mit physiologisch verträglichen Säuren überführt.

2. Verfahren zur Herstellung der Verbindungen der Formel I gemäß Anspruch 1, worin $R^2$ und $R^3$ zusammen die Gruppe $-CH_2-O-CH_2$ bedeuten und worin ein Wasserstoffatom durch eine Hydroxygruppe ersetzt ist, dadurch gekennzeichnet, daß man eine Verbindung der Formel VIIIa oder VIIIb

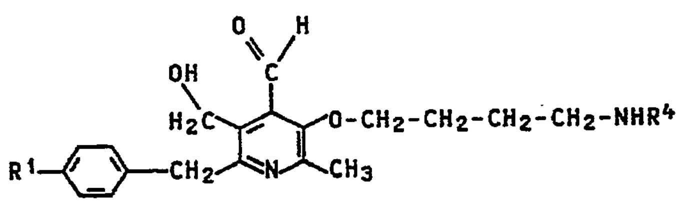

VIIIa,

oder

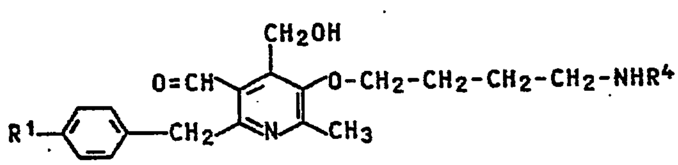

VIIIb,

worin $R^1$ und $R^4$ die angegebene Bedeutungen besitzen, in Gegenwart einer Säure umsetzt, und die so erhaltenen Verbindungen gegebenenfalls in ihre Salze mit physiologisch verträglichen Säuren überführt..

3. Verfahren gemäß Anspruch 1 oder 2, dadurch gekennzeichnet, daß man 1-Hydroxy-4-benzyl-6-methyl-7-(4-isopropylaminobutoxy)-1,3-dihydrofuro-[3,4-C]-pyridin-dihydrochlorid herstellt.

4. Verfahren gemäß Anspruch 1 oder 2, dadurch gekennzeichnet, daß man 3-Hydroxy-4-benzyl-6-methyl-7-(4-isopropylaminobutoxy)-1,3-dihydrofuro-[3,4-C]-pyridin herstellt.

**Claims for the Contracting States: BE, CH, DE, FR, GB, IT, LI, NL, SE**

1. A pyridine derivative of the formula I

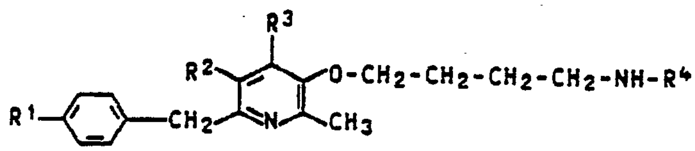

I

where $R^1$ is hydrogen or hydroxyl, $R^2$ and $R^3$ together form the group $-CH_2-O-CH_2-$ where a hydrogen atom is replaced by hydroxyl or methoxy, and $R^4$ is n-propyl, isopropyl or n-butyl, and its salts with physiologically tolerated acids.

2. 1-Hydroxy-4-benzyl-6-methyl-7-(4-isopropylaminobutoxy)-1,3-dihydrofuro[3,4-C]pyridine dihydrochloride.

3. 3-Hydroxy-4-benzyl-6-methyl-7-(4-isopropylaminobutoxy)-1,3-dihydrofuro[3,4-C]pyridine.

4. A compound of the formula I as claimed in claim 1 for use in the treatment of disorders.

5. Use of a compound of the formula I for the preparation of drugs possessing an antiarrhythmic action.

**Claims for the Contracting State: AT**

1. A process for the preparation of a pyridine derivative of the formula I

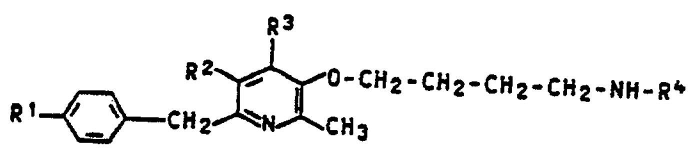

I

where $R^1$ is hydrogen or hydroxyl, $R^2$ and $R^3$ together form the group $-CH_2-O-CH_2-$, where a hydrogen atom is replaced by hydroxyl or methoxy, and $R^4$ is n-propyl, isopropyl or n-butyl, and its salts with physiologically tolerated acids, wherein

a) a pyridinol of the formula II

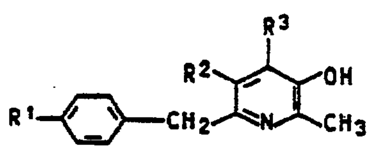

II

where $R^1$, $R^2$ and $R^3$ have the stated meanings, is reacted with a compound of the formula III

$X-CH_2-CH_2-CH_2-CH_2-NHR^4$ III

where $R^4$ has the stated meanings and X is a reactive group, or

b) a pyridinol of the formula II is reacted with a compound of the formula IV

$Y-CH_2-CH_2-CH_2-CH_2-Z$ IV

where Y and Z are each halogen, and the product is then reacted with an amine of the formula V

$NH_2-R^4$ V

where $R^4$ has the stated meanings, or

c) an amine of the formula VI

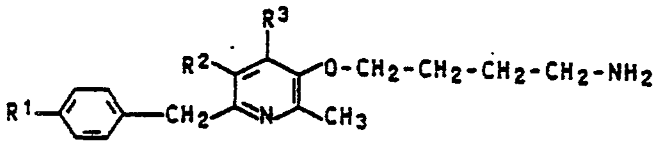

VI

where $R^1$, $R^2$ and $R^3$ have the stated meanings, is reacted with a compound of the formula VII,
$X-R^4$ VII
where X and $R^4$ have the stated meanings, and the compound thus obtained is, if desired converted to its salts with physiologically tolerated acids.

2. A process for the preparation of the compounds of the formula I as claimed in claim 1, where $R^2$ and $R^3$ together form the group $-CH_2-O-CH_2$ and in which a hydrogen atom is replaced by hydroxyl, wherein a compound of the formula VIIIa or VIIIb

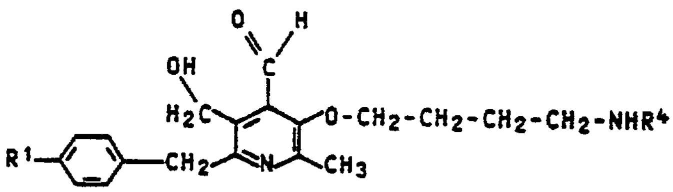

VIIIa

or

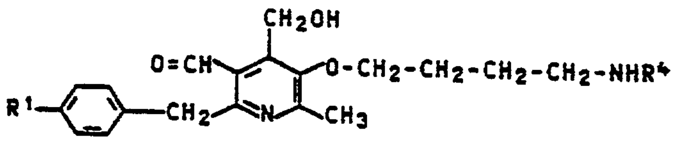

VIIIb

where $R^1$ and $R^4$ have the stated meanings, is converted in the presence of an acid and the compound thus obtained is, if desired, converted to its salts with physiologically tolerated acids.

3. A process as claimed in claim 1 or 2, wherein 1-hydroxy-4-benzyl-6-methyl-7-(4-isopropylamino-butoxy)-1,3-dihydrofuro[3,4-C]pyridine dihydrochloride is prepared.

4. A process as claimed in claim 1 or 2, wherein 3-hydroxy-4-benzyl-6-methyl-7-(4-isopropylamino-butoxy)-1,3-dihydrofuro[3,4-C]pyridine is prepared.

**Revendications pour les Etats contractants: BE, CH, DE, FR, GB, IT, LI, NL, SE**

1. Dérivés de pyridine de formule I

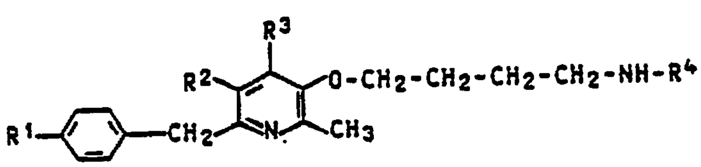

I.,

dans laquelle
$R^1$ représente un atome d'hydrogène ou un groupe hydroxy,
$R^2$ et $R^3$, ensemble, le groupe $-CH_2-O-CH_2-$ dans lequel un atome d'hydrogène peut être remplacé par un groupe hydroxy ou méthoxy, et
$R^4$, un groupe n-propyle, isopropyle ou n-butyle, ainsi que leurs sels d'acides physiologiquement acceptables.

2. Dichlorhydrate de 1-hydroxy-4-benzyl-6-méthyl-7-(4-isopropylaminobutoxy)-1,3-dihydrofuro-[3,4-C]-pyridine.

3. 3-hydroxy-4-benzyl-6-méthyl-7-(4-isopropylaminobutoxy)-1,3-dihydrofuro-[3,4-C]-pyridine.

4. Composés de formule I selon la revendication 1 pour l'utilisation dans la lutte contre des maladies.

5. Utilisation des composés de formule I pour la préparation de médicaments à effet antiarythmique.

**Revendications pour l'Etat contractant: AT**

1. Procédé de préparation de dérivés de pyridine de formule I

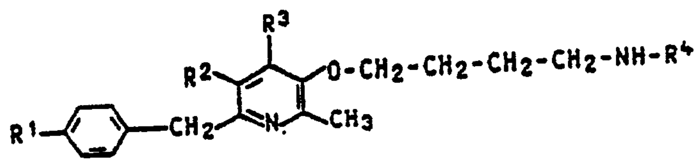

I.

dans laquelle
$R^1$ représente un atome d'hydrogène ou un groupe hydroxy,
$R^2$ et $R^3$, ensemble, le groupe $-CH_2-O-CH_2-$, dans lequel un atome d'hydrogène peut être remplacé par un groupe hydroxy ou méthoxy, et
$R^4$, un groupe n-propyle, isopropyle ou n-butyle, ainsi que leurs sels d'acides physiologiquement acceptables, caractérisé par le fait qu'on fait réagir
a) un pyridinol de formule II

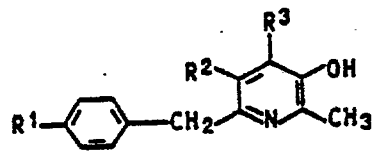

II.

où $R^1$, $R^2$ et $R^3$ ont les significations sus-indiquées, avec un composé de formule III
$X-CH_2-CH_2-CH_2-CH_2-NHR^4$ II,
où $R^4$ a la signification sus-indiquée et X représente un groupe réactif, ou
b) un pyridinol de formule II avec un composé de formule IV
$Y-CH_2-CH_2-CH_2-CH_2-Z$ IV,
où Y et Z représentent un atome d'halogène et ensuite avec une amine de formule V
$NH_2-R^4$ V,
où $R^4$ a la signification sus-indiquée, ou
c) une amine de formule VI

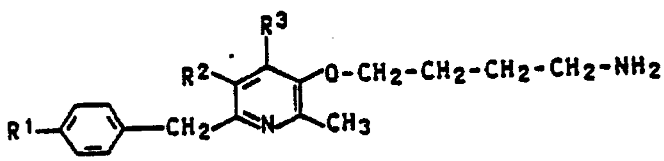

VI.

où $R^1$, $R^2$ et $R^3$ ont les significations sus-indiquées, avec un composé de formule VII
$X-R^4$ (VII)
où X et $R^4$ ont les significations sus-indiquées et on transforme éventuellement les composés ainsi obtenus en leurs sels d'acides acceptables physiologiquement.

2. Procédé de préparation des composés de formule I selon la revendication 1, dans laquelle $R^2$ et $R^3$ représentent ensemble le groupe $-CH_2-O-CH_2$ et dans laquelle un atome d'hydrogène est remplacé par un groupe hydroxy, caractérisé par le fait que l'on fait réagir, en présence d'un acide, un composé de formule VIIIa ou VIIIb

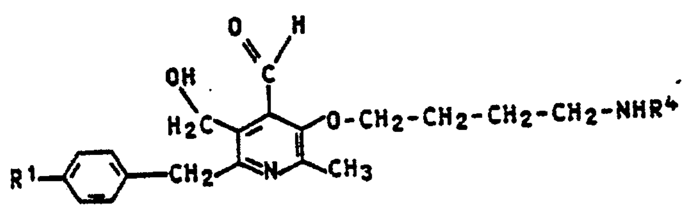

VIIIa,

ou

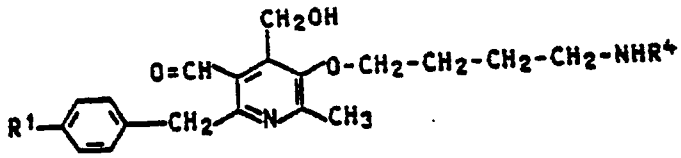

VIIIb.

où $R^1$ et $R^4$ ont les significations sus-indiquées et on transforme éventuellement les composés ainsi obtenus en leurs sels d'acides acceptables physiologiquement.

3. Procédé selon la revendication 1 ou 2, caractérisé par le fait que l'on prépare du dichlorhydrate de 1 hydroxy-4-benzyl-6-méthyl-7-(4-isopropylaminobutoxy)-1,3-dihydrofuro-[3,4-C]-pyridine.

4. Procédé selon la revendication 1 ou 2, caractérisé par le fait que l'on prépare du 3-hydroxy-4-benzyl-6-méthyl-7-(4-isopropylaminobutoxy)-1,3-dihydrofuro-[3,4-C]-pyridine.